# EUROPEAN PATENT SPECIFICATION

(11) **EP 0 472 622 B1**
(45) Date of publication and mention of the grant of the patent: **13.12.1995**
(21) Application number: 90908395.8
(22) Date of filing: 14.05.1990
(51) Int. Cl.: C12M 1/34, G01N 21/78

(54) **APPARATUS FOR DETECTION OF MICROORGANISMS**
VORRICHTUNG ZUM NACHWEIS VON MIKROORGANISMEN
APPAREIL DE DETECTION DE MICRO-ORGANISMES

(30) Priority: 15.05.1989 US 351476
(43) Date of publication of application: 04.03.1992
(73) Proprietor: Akzo Nobel N.V., 6824 BM Arnhem (NL)
(72) Inventor: DIGUISEPPI, James, L., Durham, NC 27712 (US); THORPE, Thurman, C., Durham, NC 27704 (US)
(74) Representative: Hermans, Franciscus G.M.
(86) International application number: US9002631
(87) International publication number: WO9014414

(56) References cited:
- EP-A- 301 699
- EP-A- 333 253
- US-A- 4 101 383
- US-A- 4 456 380
- PATENT ABSTRACTS OF JAPAN, vol. 10, no. 351 (P-520)(2407), 27 November 1986/

## Description

### BACKGROUND OF THE INVENTION

The present invention relates to methods and devices for detection of bacterial growth within a specimen. More particularly, the present invention relates to an electronic apparatus for detecting microorganism growth within a sample through monitoring of an indicating element exposed to the sample growth.

The presence of microbial contamination in a clinical specimen is conventionally determined by culturing the specimen in the presence of nutrients and detecting microbial activity through changes in the specimen or in the atmosphere over the specimen after a period of time. For example, in U.S. Patent 4,182,656, to Ahnel, et al., the sample is placed in a container with a culture medium comprising a Carbon 13 fermentable substrate. After sealing the container and subjecting the specimen to conditions conducive to biological activity, the ratio of Carbon 13 to known Carbon 12 in the gaseous atmosphere over the specimen is determined and compared with the initial ratio. In U.S. Patent 4,152,213, a method is claimed by which the presence of oxygen consuming bacteria in a specimen is determined in a sealed container by detecting a reduction in the amount of oxygen in the atmosphere over the specimen through monitoring the gas in the container. U.S. Patent 4,073,691 provides a method for determining the presence of biologically active agents, including bacteria, in a sealed container, containing a culture medium by measuring changes in the character of the gaseous atmosphere over the specimen after a period of time.

A method for non-invasive detection of CO₂ changes in the gaseous atmosphere is taught by Suppman, et al. as disclosed in EPO Application 104463, published April 4th, 1984. The methods and apparatus described in these and other publications all require either a radiometric method or the invasion of the sealed container to measure changes in the gaseous atmosphere after culturing or require that the container be manufactured of special materials that permit unimpeded passage of infrared light.

EP-A-0 301 699 refers to the detection of microbial growth in a specimen in conventional microtiter plates. The detection of metabolic activity is based on the measurement of colour changes, or other properties affecting the reflectance of the sample, in the sample itself.

US-A-4 456 380 discloses a method for the identification of bacteria in a sample in conventional culture plates based upon specific colour reactions of the bacteria with reagents contained in the single cells of the plates. The method does not produce a quantitative signal.

Other known methods for measuring microbial contamination of a specimen, particularly blood cultures, include measuring minute changes in temperature, pH, turbidity, color, bioluminescence, and impedance. Generally, these methods determine microbial contamination by detecting bacterial metabolic by-products. Microbial contamination may also be assessed by subculturing and/or staining. Of these methods, only impedance, radiometry, and infrared spectrometry provide the possibility of automated processing of a clinical specimen. Except for impedance and infrared measurements, these procedures also require entering the container in order to make a measurement on the liquid specimen or the gaseous atmosphere over the specimen.

In addition to the likelihood of contamination and creating the likelihood of altering the constituency of the atmosphere over the specimen each time the determination is made, these methods do not permit taking measurements continuously or easily over short-time intervals for an extended period of time. This is a significant disadvantage as the rate of growth of contaminating organisms differs, depending on the organism and the number of organisms in the original sample, such that it cannot be predicted when detectable changes in the atmosphere or fluid sample will be presented by a contaminated specimen.

In a related problem, when contamination is determined by pH changes in the liquid sample, various metabolic products will effect the pH of the sample differently. For example, the production of ammonia will raise the pH, while the production of CO₂ will lower it. Different growth rates of different contaminating organisms could result in a pH increase at one time and a decrease at another time. This variable increase-decrease would not be detected if the pH is measured at widely-spaced intervals. Another source of error when detecting changes by pH measurement in whole blood samples, particularly when an indicator is the means for pH determination, is the likelihood that the appearance of the indicator can be affected or obscured by the presence of blood cells. Colorimetric indicators can only be effectively used if errors caused by the nature of the specimen can be prevented from influencing the appearance of the dye.

### SUMMARY OF THE INVENTION

It is an object of the present invention to provide an apparatus for continuous monitoring of changes in the pH or CO₂ levels within a specimen containing a culture for microbial growth.

It is another object of the present invention to provide an apparatus for monitoring pH or CO₂ changes in a specimen within a sealed container, without entering the container during the monitoring process.

It is a further object of the present invention to provide a pH and/or CO₂ indicator which provides continuous indication of pH or CO₂ levels and is unaffected by the presence of colored constituents within the growth medium.

Another object of the present invention is to provide analysis of the variance in pH and/or CO₂ on a continuously monitored basis.

A further object of the present invention is to monitor both the absolute pH value and the rate of change of pH. These and other objects of the present invention are accomplished by providing an apparatus for detecting the presence of microorganisms in clinical specimen, such as blood or other body fluids, by culturing the specimen with a sterile growth medium in a transparent, sealed, sterile container. The presence of microorganisms is determined by detecting or measuring changes in the pH of the specimen or the production of CO₂ within a specimen using a disposable sensor affixed to the interior surface of the container. According to this invention, microorganisms can be detected in the presence of interfering materials, such as large concentrations of red blood cells, through non-radiometric and non-invasive means. As the level of pH and/or CO₂ within the specimen changes, the light reflecting and/or absorbing characteristics of the disposable sensor will alter correspondingly. The quantity of alteration of the reflective properties of the sensor is detected by an emission and receiving mechanism which supplies signals to a device for monitoring the quantity of visible reflection/absorption and the rate of change. The rate and quantity is then analyzed to predict and determine the presence of microbial growth within the specimen or sample. The sensor can be sampled and/or monitored continuously or at frequent time intervals allowing for the collection of a detailed characteristic of the quantity and rate of sensor change.

A more complete appreciation of the invention and many of the attendant advantages thereof will be readily perceived as the same becomes better understood by reference to the following detailed description when considered in connection with the accompanying figures.

### BRIEF DESCRIPTION OF THE DRAWINGS

Figure 1 is a cross-sectional view of the specimen jar and photodetector arrangement.

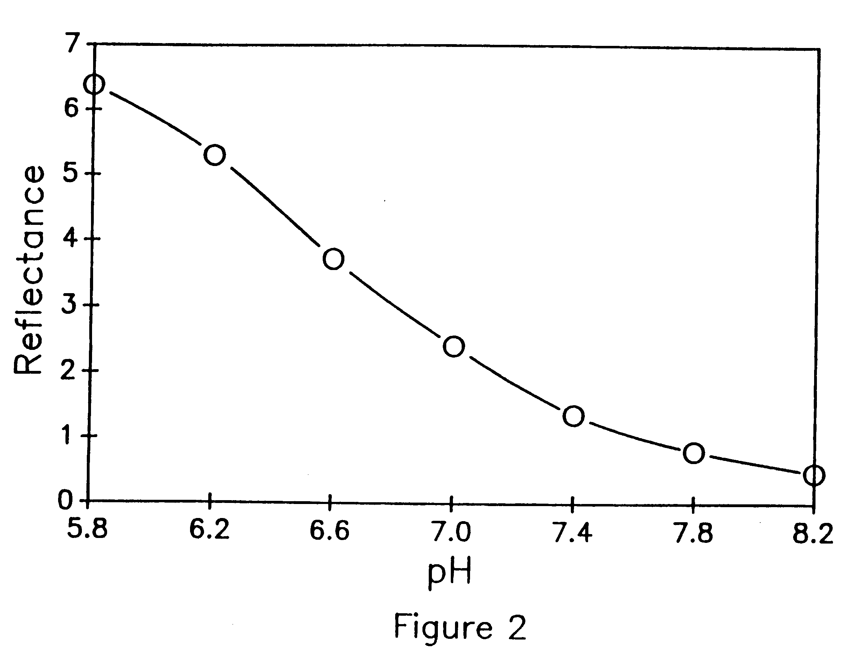

Figure 2 is a graph showing the characteristic relation between reflectance of the sensor and the pH of the sample.

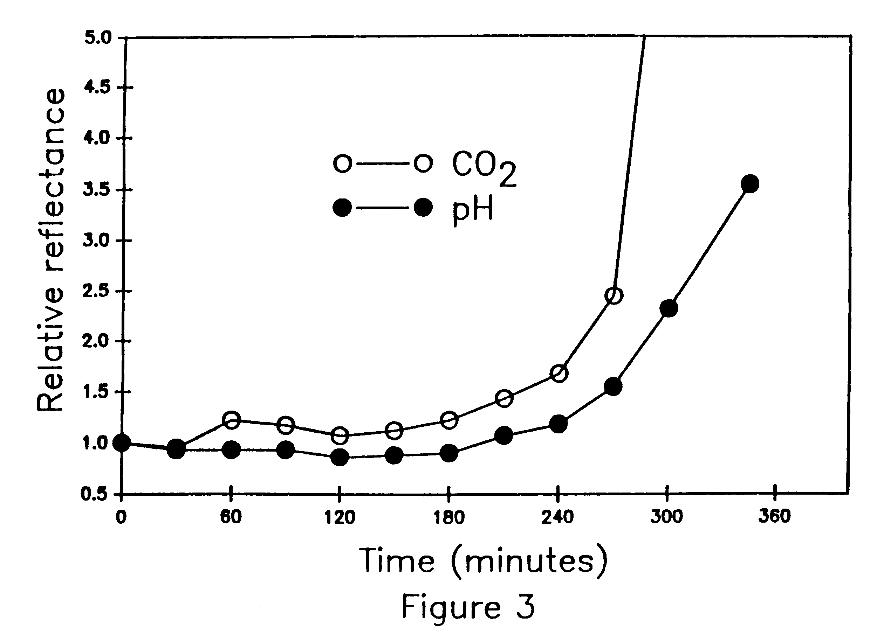

Figure 3 is a graph illustrating a characteristic which is exemplative of the pH and CO₂ changes in a sample caused by the growth of E. coli bacteria.

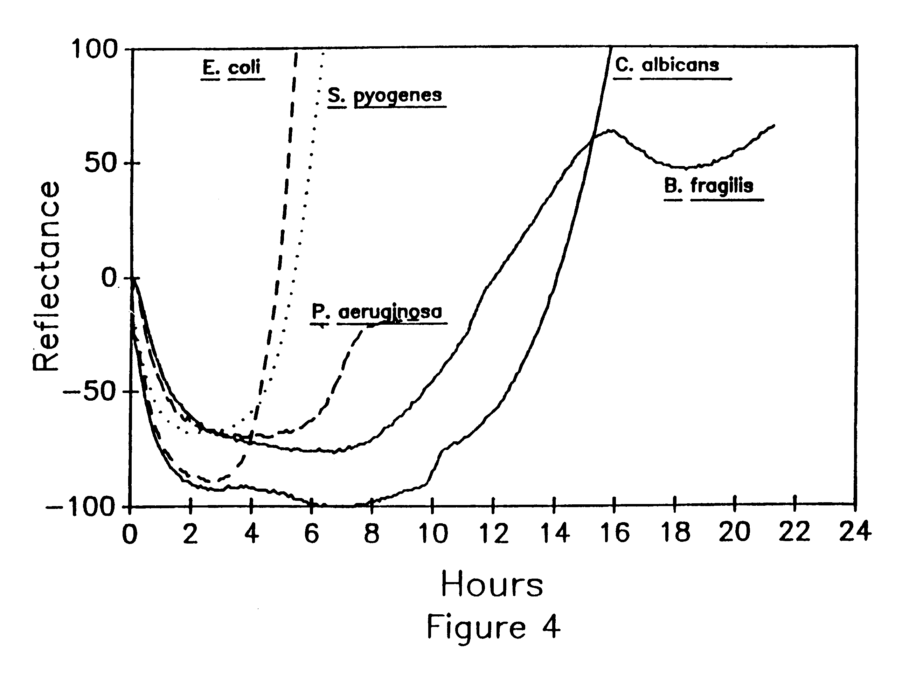

Figure 4 is a graph illustrating the change in reflectance of a variety of microorganisms over time as measured by the present invention.

Figure 5 is a graph illustrating the threshold rate generation level versus constant slope monitoring detection methods.

Figure 6 is a schematic block diagram illustrating the major components of the analysis apparatus of the present invention.

Figure 7 is a perspective view of an embodiment of the present invention illustrating eight simultaneously monitored samples.

### DESCRIPTION OF EXEMPLARY EMBODIMENTS

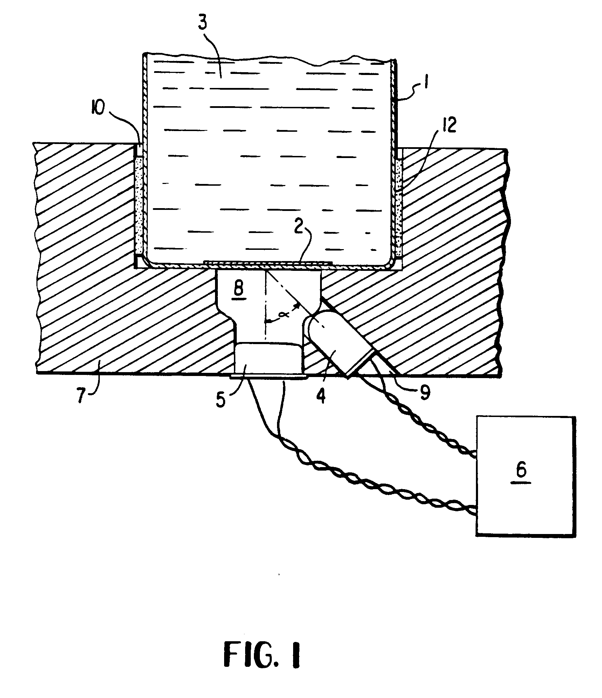

The apparatus and device of the invention provide a non-invasive means for detecting the presence of microorganisms in a clinical specimen such as blood samples or other bodily fluids, by measuring an increase in metabolic products produced by microorganisms. A specimen is added to a specially-formulated medium that enhances the production of certain microbial metabolic products. As illustrated in Figure 1, the specimen and the formulated medium 3 are housed in a culture bottle or container 1 for incubation. The container 1 is sealed by a top not illustrated.

A unique disposable sensor or indicator 2 is located within the bottle 1 positioned against an interior wall thereof. The sensor 2 comprises a solid composition or membrane which is referred to as the attachment or support medium and an indicator medium mounted on or within the support medium. In this manner, the sensor 2 is placed flush against the inside surface of a container 1 such that the indicator medium is visible from the outside, and is sealed against cells, proteins or other solids or opaque or colored components of the specimen or medium from getting between the indicator 2 and the container 1 surface. This will prevent any objects from coming between the indicator 2 and the detector mechanism 4 and 5. In certain embodiments, the indicator 2 is separated from the specimen and its growth medium 3 by a membrane or solid layer that permits the passage of gas molecules, but prevents the passage of ions. In this manner, the indicator 2 will be exposed to changes in pH or CO₂ as desired, but will not be directly exposed to the medium 3.

Specimens of body fluids such as blood, containing as few as one organism per milliliter can be detected using this invention. Such specimen require up to seven days incubation before the population of organisms reaches a critical level, or an increase in metabolic products can be measured. It has been found through operation of the present invention that a concentration of 10⁶ CFU/ml for certain types of organisms provided measurable levels in pH or CO₂. All organisms showed measurable results at concentrations of 10⁷ to 10⁸ CFU/ml.

The invention is unique and advantageous in several respects:
(1) the microbial metabolic products are measured in the liquid phase of the culture bottle rather than in the atmosphere over the specimen,
(2) the unique disposable sensor 2 being affixed to the interior surface of the bottle 1, measurements can be made from the outside the transparent wall of the bottle 1 without having to violate the integrity of the bottle 1,
(3) the external measurements can be made by visual inspection or with an instrument that measures by measuring the reflectance of the sensor 2,
(4) opaque or colored components in the specimen 3 do not interfere with the ability of the sensor 2 to detect and indicate changes in the pH and or CO₂ levels, and
(5) a high concentration of indicator molecules is maintained within a small volume, i.e., on the membrane, such that a color change can be easily observed,
(6) observation of the sensor 2 can be affected essentially continuously through sampling over closely-spaced time intervals since there is no invasive sampling or vessel manipulation required,
(7) a characteristic representative of the absolute level and rate of change of pH or CO₂ can be developed from the continuous monitoring.

The nutritional components that make up a complex microbial medium influence the metabolic pathways used by microorganisms. Organic acids, bases and various gases are produced in proportion dependent upon the nutrients available. These products also vary from species-to-species of microorganisms. The presence of these products in the liquid medium can change its pH. Sensors used in the invention contain pH sensitive indicators that give a measurable change in response to a pH change in the environment. In the embodiment in which the pH sensor is covered by a gas permeable, ion impermeable membrane, the presence of gases that effect the pH of the indicator, such as CO₂ or ammonia, are measured. Thus, microbial growth can be detected either by changes in pH of the liquid culture medium or by measurement of gases dissolved in a medium produced by microorganisms. Carbon dioxide is a universal metabolite produced by all organisms and, therefore, is the preferred metabolite for detection of microbial growth.

CO₂ and pH sensors as taught in the present invention share two common components, a molecular species useful as a pH indicator and an attachment/support medium. The pH indicator can be attached either covalently or non-covalently to the support medium. Alternatively, the indicator can be encapsulated within a polymer matrix that is gas permeable such as a pH indicator emulsified within a polymer matrix prior to curing. The CO₂ sensor has a third component, a semi-permeable substance that completely separates the indicator membrane from the specimen and growth medium. These sensors are fixed inside a suitable transparent vessel 1 with an appropriate adhesive.

A variety of different fluorescent and visible pH indicators can be used as the active molecular species in pH or CO₂ sensors. Generally, the only limitations on the selection of indicators are the requirements that they have acceptable dynamic pH ranges and exhibit wavelength changes that are readily detectable by existing front surface fluorescence or reflectance technologies.

Sensors for detecting pH changes in the culture medium according to the invention should exhibit a change in fluorescence intensity or visible color over a pH range of about 5.0 to about 8.0.

Indicators for a CO₂ sensor should exhibit a change in fluorescence intensity or visible color in a pH range between about 10.0 and 6.0 in order to detect changes in CO₂ concentrations.

Only certain pH indicator molecules can be bound covalently or non-covalently to a support medium and retain their pH indicating properties. We found indicators belonging to the xanthene, phenolphthalein and phenolsulfonphthalein groups to be useful.

The attachment/support medium can be a substance such as cellulose, to which a pH indicator can be covalently attached using organic reactions. Non-covalent attachment of pH indicators can be achieved using ionic support materials, such as nylon membranes that have a positive or negative zeta potential. Other ionic support materials that can be used are positively or negatively charged ionic resins, such as diethylamino ethyl (DEAE) resin or (DEAE) cellulose. Pretreatment of the support material with a protein may be required if the indicator membrane is to be in direct contact with the microbial growth media.

The pH indicator sensors directly detect pH changes due to the pH environment of the microbial growth medium. However, these sensors can be made to selectively react to gases (e.g., carbon dioxide or ammonia) in the liquid growth medium by covering them with a selectively semi-permeable composition or membrane such as silicon, latex, teflon or various plastics characterized by the capacity to selectively permit the diffusion of a gas while preventing the passage of ions and liquid. For sensors comprising an indicator encapsulated within a polymer matrix, the polymer forming the matrix can act as the semi-permeable barrier that permits the passage of gases, but not ions.

In the encapsulated indicator embodiment the CO₂ sensor is comprised of a visual or fluorescent pH indicator which is reactive in the pH range between 6 pH and 10 pH, a sodium hydroxide or an equivalent base which maintains an optimal pH environment for detection of CO₂, a glycerol or equivalent emulsifier which can produce micelles of indicator solution emulsified within the uncured polymer and an uncured polymer such as room temperature RTV white silicon which maintains the proper environment for the indicator. Any polymer can be used that does not affect the chemical activity of the indicator, either from its own chemical or physical properties or its requirements for curing as long as it is permeable to gases but not to ions and does not have these properties altered when subjected to sterilization by autoclaving.

An emulsion is prepared from the four components above and the polymer is cured to form a semi-permeable matrix around the micelles of pH indicator, which permits selective diffusion of CO₂ and other gases from the liquid microbial growth medium, resulting in a measurable exposure of the indicator to provide a measurable visual change in the indicator color. The sensor 2 can be prepared separately and adhered to the interior surface of the culture bottle or, in the alternative, the sensor 2 can be formed on the bottom of the bottle and cured in situ. After curing, the bottle with the sensor is sterilized, such as by autoclaving. A number of specific examples of pH and CO₂ sensors are provided in co-pending U.S. Patent US 4945060, filed March 15th, 1988. Specific pH and CO₂ indicators are not herein detailed beyond the above description. The sensors must provide visually detectable indication of pH and or CO₂ changes in order to properly interact with the instrument and equipment described herein below.

Similarly, culture medium formulations are described in the US patent No. US 4945060 and are therefore not detailed herein.

Figure 1 illustrates a cross-sectional view of a culture container 1 with a sample and medium 3 therein and a sensor 2 attached to the bottom surface thereof. The container 1 is illustrated situated on a platform 7 which has an opening 8 into which is inserted photodetector 5 and an opening 9 into which is inserted emitter 4.

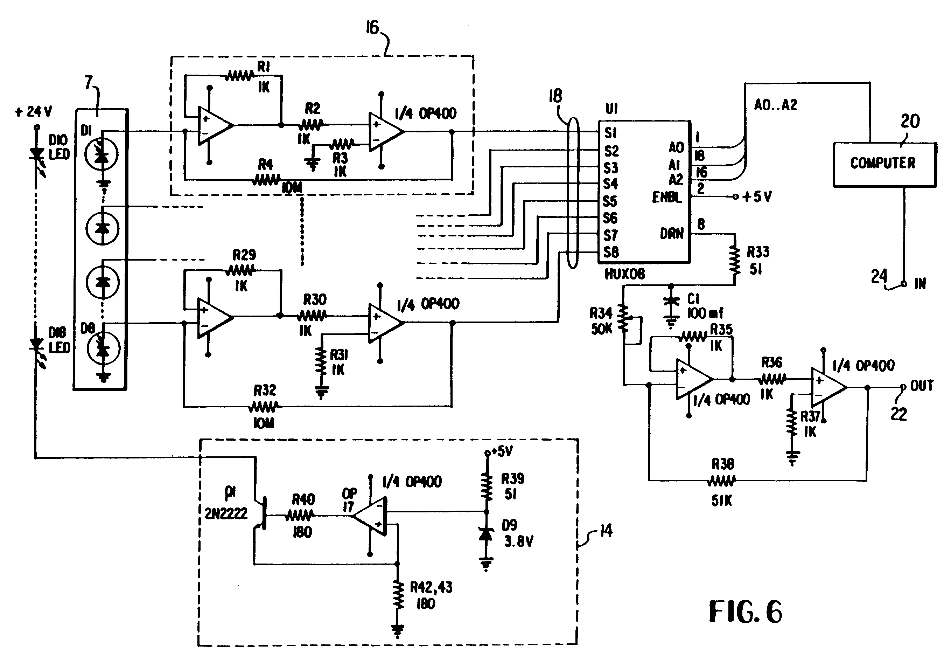

Both the emitter 4 and the detector 5 are connected to the analysis apparatus 6 illustrated in greater detail in figure 6.

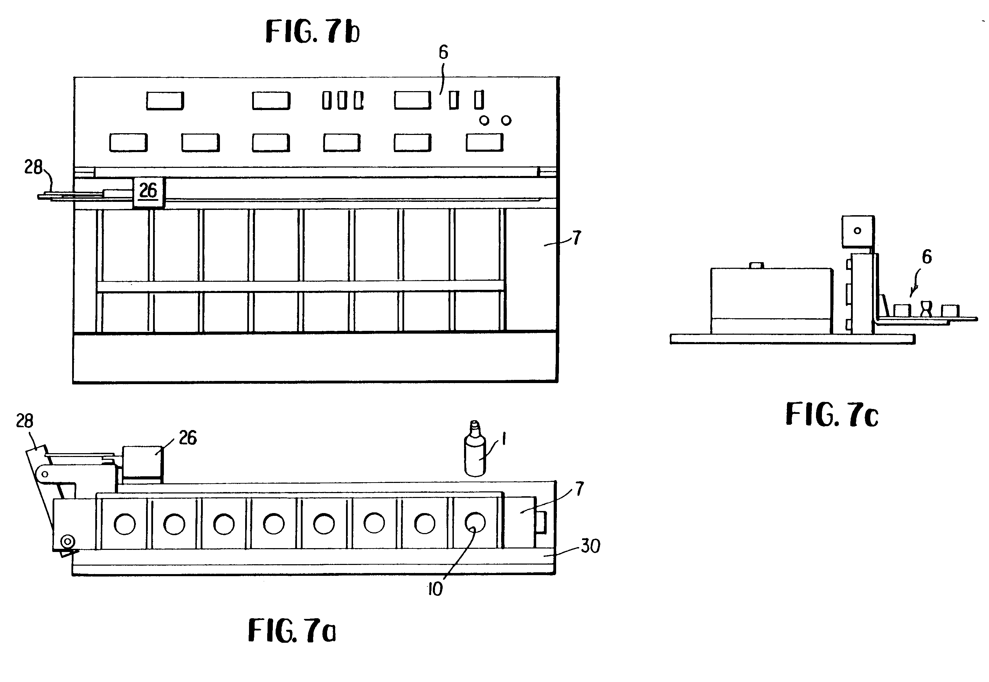

Containers 1 are generally cylindrical in form, as illustrated in Figure 7, and fit into openings 10 in the upper surface of substrate 7. The containers 1 are maintained snugly within the openings 10 through use of resilient packing material 12. The interior of the openings 10 are lined with the packing material 12 which resiliently presses against the sidewalls of container 1, when it is fitted into the openings. This packing is provided to prevent the containers 1 from falling out of the openings 10 during motion of the substrate 7 as described below. Emitter 4 and detector 5 are mounted at a relative angle α to provide for optimal reception of reflected light from indicator 2. Angle α in the illustrated preferred embodiment is 45 degrees, however, other relative angles near 45 degrees provide acceptable results. A preferred range of 30 to 60 degrees has been found optimal to give the best reflective efficiency.

The emitter 4 and detector 5 must be placed at an appropriate relative angle and at an appropriate angle relative to the indicator 2, so that the light will not reflect off the exterior of container 1 but will reach indicator 2 to reflect therefrom. Further, the detector 5 must be shielded from direct illumination from the emitter 4 to prevent an inaccurate reading.

Figure 7 illustrates the overall configuration of one exemplary embodiment of the present invention. As can be seen in Figure 7, the base member 7 has a number of cylindrical slots holes 10 to accommodate the sample bottles 1. Any number of bottles 1 can be placed into the slots 10 in the substrate or base 7. The base 7 is mounted in a manner that it can be rocked by rocking motor 26 and rocking mechanism 28 attached thereto. The rocking is necessary to keep the mixture properly agitated and stirred within the bottles 1 to promote proper microbacterial growth. The base is also heated by means of heaters 30 which are secured to the base or by total enclosure of the base in a controlled incubation apparatus. Each of the slots 10 includes an emitter and a detector for appropriately illuminating the sensors and detecting the reflected luminescence.

As detailed below, each of the bottles 1 can be independently monitored through selected reading of the outputs of the detectors associated with the slots into which each of the bottles have been inserted. In this manner, computer 20 can monitor a number of samples simultaneously taking a sample reading of each sample as desired at a pre-determined desired sampling rate and evaluating the microbacterial growth progress of each sample as detailed herein below.

Figure 6 is a circuit diagram illustrating the signal reception and processing portion of the signal analysis device 6 illustrated in Figures 1 and 7. The LED's D11 through D18 are powered by a Zener stabilized constant current source 14. The current source includes transistor Q1 operational amp op17 and Zener diode D9. The current source 14 provides a stable illumination for the LED's 11 through 18 illuminating diode receptors D1 through D8.

LED's D10 through D18 are representative of the light emitter 4 illustrated in Figure 1 and photo diodes D1 through D8 are illustrative of the light detector 5 illustrated in Figure 1.

In Figures 6 and 7 there are eight diodes mounted on substrate 7, four of which are illustrated. The exemplary embodiment illustrates eight light emitters, eight detectors and eight parallel circuitry paths, this is for exemplary purposes only and any number of emitters and detector pairs can be utilized dependent upon the number of samples to be evaluated by a single processing apparatus 6.

The photo diodes D1 through D8 illustrated can, for example, be VTP 5051 photo diodes from EGG Vactech of St. Louis, Missouri. Any comparable photo diode capable of sensitive detection in a given area of the spectrum can be utilized. Other photo diodes capable of detection in other areas of the electromagnetic spectrum can be utilized if the photo emitters 4 are so designed to emit in that portion of the spectrum and the sensor 2 is designed to exhibit reflective properties in that different portion of the spectrum in response to changes in pH or CO₂ production.

The output of each diode is amplified by a pair of low offset operational amplifiers arranged in a low drive inverting configuration. This configuration is illustrated by that portion of the circuit identified as 16, in Figure 6.

The outputs of the operational amplifiers are multiplexed into multiplexer MUX08 on individual input lines 18. In this manner the output of any one of the individual diodes D1 through D8 can be selected for monitoring and or analysis. Through provision of a three bit address provided on address lines A0 through A2 the computer 20 can select the desired diode output to be read out on DRN line 8 from multiplexer MUX08. The signal out from the multiplexer is then passively low pass filtered and amplified and provided as an analog signal output for computer 20. This analog signal at the output terminal 22 can first be converted to a digital signal and then passed to the input terminal 24 of the computer, or can be passed directly to the computer as a analog signal.

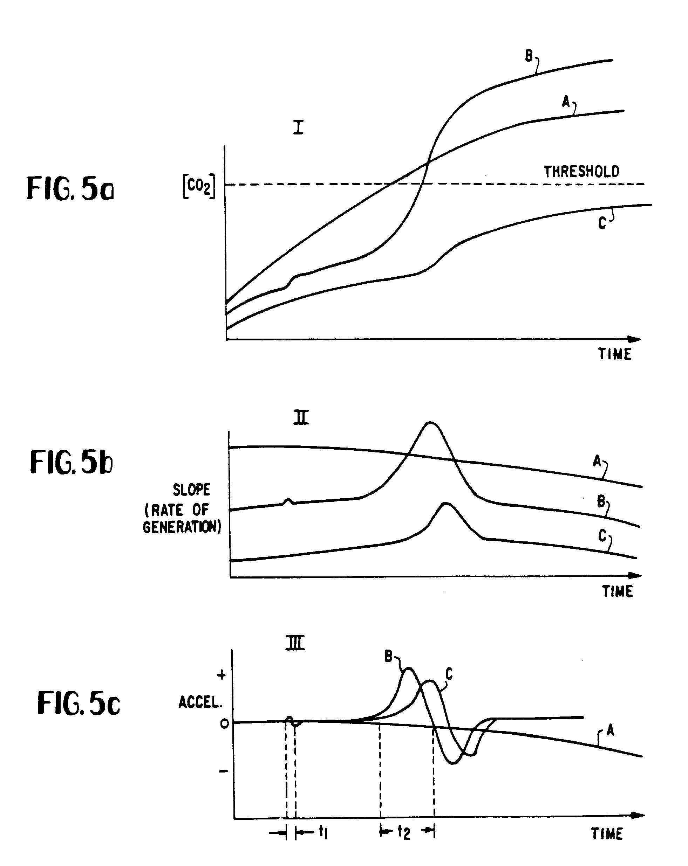

Within the computer 20, the outputs of the various detectors are compiled, and the curves characteristic of the quantity and rate of change of pH or CO₂ concentration of the various samples and illustrated in Figure 5 are developed. The computer also performs the necessary analysis to evaluate the characteristics developed and to determine the presence or non-presence of developing microbial cultures as described herein below.

With reference to the graphs of Figure 5, the advantages of the analysis technique available through the present invention over those analysis techniques available in the prior art or through visual inspection are described below.

In the first graph I of Figure 5, the absolute level of CO₂ has been plotted against time, along with a threshold CO₂ level which might be used to determine if microbial contamination was present. The absolute CO₂ concentration value of the solution is determined through analysis of the color change of the indicator 2 within the container 1. It should be noted that bodily fluids such as blood demonstrate a production of CO₂ of their own, which is seen in the steadily rising levels in all three curves. The magnitude of this background production varies greatly among specimens. Characteristics A, B and C represent exemplary microbial growths. Characteristic A illustrates a high level of background production, but no microbial growth. It crosses the threshold and would therefore be considered a positive under prior art analysis techniques, but would be a false positive. Characteristic B illustrates a moderate background level and strongly visible growth. Since characteristic B also crosses the threshold level, it would be detected as true positive. These can be detected by periodic sampling techniques having a significant elapsed time between sampling because the CO₂ level remains above the threshold for a significant period of time. Characteristic C illustrates a combination of low background production and poor growth, resulting in a false negative under prior detection analysis.

The second graph II illustrates the first derivative of the data in the first graph, or the rate of CO₂ production. It can be seen that the rate of A is high but constantly decreasing; of B moderate with an increase at the time of microbial growth; and C low, but again with an increase at the time of microbial growth.

The third graph III illustrates the second derivative, or growth acceleration, of the data in the first graph. Here, characteristic A illustrates a constantly negative acceleration, whereas B and C show periods of strongly positive acceleration. In comparison of the graphs, it can be seen that, although the CO₂ value of the sample A rises above a threshold value in graph I, it does not exhibit a significant slope change in graph II and therefore no acceleration in graph III. It can be seen that in graph III the two true positive samples B and C exhibit positive characteristics not present in sample A.

The characteristic C illustrates a microbial growth exhibiting a slower CO₂ generation rate which can be due to a variety of factors. In this instance, through use of the sampling techniques of the prior art or through visual inspection of the indicator 2 of the present invention, this sample would be determined to be a negative which would be a false negative. This is due to the fact that the absolute CO₂ value of sample C never rises above a threshold value necessary to indicate a positive reading. However, if the sampling, analysis and evaluation technique of the present invention described below is utilized, this actual microbacterial growth, although not sufficient to raise the CO₂ of the sample above at threshold level in graph I, would be detected as a positive bacterial presence and therefore a true positive.

The computer 20 of the present invention can be set to read the indicated CO₂ value at any given interval. For example, when we use a sampling interval of ten minutes between readings, the computer will instruct the multiplexer MUX08 to output a reading from the appropriate sensor input at ten minute intervals. The reading will be read and recorded by the computer. The computer then takes another reading after ten minutes and records the value.

After six intervals have been gained, a seventh interval is taken and the first interval is dropped. The computer will continue to update in this manner by dropping the oldest sample and adding the most recent, so that at all times the computer has a one hour period of data at hand.

The computer 20 utilizes these samples to continuously determine the slope of the curve connecting each adjacent pair of pH readings and therefore establishes an "instantaneous" slope value between each pair of adjacent ten minute interval readings. This slope is compared to the previous interval slope and to the slopes of the intervals preceding that back to a total of six intervals or an hour. This provides the computer with the rate and acceleration values represented in graphs II and III.

The slope of the present ten-minute sampling period can be compared solely against the slope of the last period or can be compared against the slope of the last five periods on a weighted basis. The computer looks for positive acceleration such as those illustrated by the curves B and C of graph III, of Figure 5. The time duration of the positive acceleration is monitored, and the acceleration must remain positive for a period of time greater than that which could be attributed to instrument fluctuations or other background interference. For example, time period t₁ in graph III is insufficient whereas time period t₂ is sufficient.

This method of analysis is advantageous in that both examples B and C which are positive bacterial growths will be detected by the present invention. This analysis scheme is not dependent upon the absolute value of the CO₂ developed within the sample, but only depends upon a change in the rate of CO₂ increase which has been found to be a more reliable indicator of positive biological samples than is the crossing of a threshold by the absolute CO₂ value.

This method of analysis is only achievable through the apparatus and teaching of the present invention which allows for continuous monitoring and close spaced sampling and which provides an indicator 2 of sufficient sensitivity to allow for high precision CO₂ readings to be obtained.

The sensors monitored by this instrument contain indicators that change color when growing or metabolizing microbes are present and generate either a pH or a CO₂ change. Although the color changes may be apparent to the naked eye, use of the instrumentation 6 provides the advantage of objectivity, sensitivity, and continuity of measurement. In the preferred embodiment, a light emitter and detector are provided for each sensor. However, other alternatives are available to allow continuous monitoring of all the samples. These alternatives include utilizing means to move each specimen bottle past one or more stationary detectors or to move one or more detectors past the stationary bottles.

In the preferred embodiment described above solid state illuminators and detectors are utilized; however, incandescent and arc lamp sources of illumination could also be used and mirrors, lenses, optical fibers and other means of directing the light to the indicator within the bottle for reflection to a detection means can also be utilized as would be apparent to the skilled artisan after the disclosure of the present invention.

## Claims

1. An instrument for monitoring microbial growth in a specimen, comprising:
a sealable, sterilizable container having an internal chamber in which the specimen is cultured in a sterile culture medium, the container having at least one transparent section;
a sterilizable indicator means located in the container in the region of the transparent section, said indicator means exhibiting a measurable change in response to a pH change in its environment detectable through said transparent section upon exposure to metabolites of microbial growth, whereby changes in the indicator means can be monitored from the exterior of the container through said transparent section, thereby monitoring microbial growth without entering the container after sealing; an emitter means for emitting an emitter signal that interacts with at least one measurable property of said indicator means whereby an indicator signal is produced, said emitter means being positioned relative to said indicator means so that said emitter signal strikes said indicator means through the transparent section;
a detector means positioned relative to said indicator means for receiving said indicator signal from said indicator means through the transparent section and for producing a detector signal corresponding thereto;
processing means for receiving said detector signal and for processing said detector signal to evaluate the magnitude of the measurable property of said indicator means at any given time and compare the magnitude of said signal with the magnitude at another time and thereby monitor microbial growth in said sealable container after said container has been sealed.

2. An instrument according to claim 1, wherein said processing means for receiving said detector signal is a circuit means.

3. An instrument according to claim 1, said emitter means comprising a light emitting diode.

4. An instrument according to claim 3, said detector means comprising a photodiode receptive to reflection of the emission of said light emitting diode.

5. An instrument according to claim 3, said processing means for receiving said detector signal comprising a stabilized current source for said light emitting diode.

6. An instrument according to claim 2, said circuit means comprising:
computing means for receiving said detector signal at selected time intervals and for comparing any changes in the characteristics of said detector signal during each of said time intervals.

7. An instrument according to claim 2, said circuit means comprising computation means for periodic sampling of said detector signal, for comparing samples of said detector signal, and for calculating the rate of change of said samples.

8. An instrument according to claim 1 further comprising means for detecting a rate of change in said detector signal and for measuring the duration of said rate of change.

9. A method for detecting microbial growth in a specimen, comprising the steps of:
providing a sterilizable container, the container having at least one transparent section in a wall thereof and an indicator means that exhibits a measurable change in response to a pH change in its environment disposed in the container in the region of the transparent section;
providing an emitter means for emitting an emitter signal that interacts with at least one measurable property of said indicator means whereby an indicator signal is produced, said emitter means being positioned relative to said indicator means so that said emitter signal strikes said indicator means through the transparent section;
providing a detector means positioned relative to said indicator means for receiving said indicator signal from said indicator means and for producing a detector signal corresponding thereto;
providing a processing means for receiving said detector signal and for processing said detector signal to evaluate the magnitude of the measurable property of said indicator means;
introducing the specimen under sterile conditions into the container;
incubating the specimen in the container;
and
detecting a change in, or the magnitude of, the detector signal thereby detecting microbial growth in the sealable bottle after the container has been sealed.

10. A method according to claim 9, comprising the further steps of:
receiving said detector signal at selected time intervals; and
comparing any changes in the characteristics of said detector signal during said time intervals.

11. A method according to claim 9, comprising the further steps of:
sampling said detector signal;
comparing successive samples of said detector signal; and
calculating the change in the rate of change between successive samples.

12. A method according to claim 9, comprising the further steps of:
detecting an increase in the measurable property; and
measuring the duration of said measurable property being at that increased level.

13. A method of determining the presence of microbes in a specimen, comprising the steps of:
providing the instrument of claim 1;
introducing the specimen into the sterile container;
incubating the specimen in the container;
monitoring for a change in a measurable property of the indicator means with the detector means at at least one time interval;
calculating the rate of change of the measurable property, whereby a first derivative of the measurement of the property is determined; and
analyzing the magnitude of the first derivative of the measurable property to establish the presence of microbes in the specimen.

14. A method of determining the presence of microbes in a specimen, comprising the steps of:
providing the instrument of claim 1;
introducing the specimen into the sterile container;
incubating the specimen in the container;
monitoring for a change in a measurable property of the indicator means with the detector means at at least one time interval; and
measuring a magnitude of the measurable property to establish the presence of microbes in the specimen.

15. A method according to claim 13, comprising the further steps of:
calculating the second derivative of the measurement of the measurable property; and
analyzing the magnitude of the second derivative of the measurable property whereby the presence of microbes in the specimen is ascertained.

16. A method according to claim 15, comprising the further steps of:
measuring the duration of said second derivative being at a certain magnitude.

17. An instrument according to claim 1, wherein the indicator means comprises a membrane and an indicator medium, the indicator medium exhibiting a detectable change in response to a pH change in its environment.

18. An instrument according to claim 1, wherein the membrane is sealed to an interior wall of the container.

19. An instrument according to claim 17, wherein the indicator medium is disposed against an interior wall of the container and the membrane of the indicator means separates the indicator medium from the culture medium.

20. An instrument set forth in claim 1, wherein the metabolite is CO₂.

21. An instrument according to claim 1, wherein the indicator medium is a chemical responsive to pH.

22. An instrument according to claim 1, further comprising means for detecting a change in the rate of change.

23. An instrument according to claim 22, also measuring the duration of said change in the rate of change.

24. An instrument according to claim 1, also measuring the duration, said detector signal being at a given magnitude.

## Patentansprüche

1. Instrument zum Ueberwachen von mikrobiellem Wachstum in einer Probe, umfassend:
einen versiegelbaren, sterilisierbaren Behälter mit einem Innenraum, in dem die Probe in einem sterilen Nährmedium kultiviert wird, wobei der Behälter mindestens einen transparentan Abschnitt aufweist;
ein sterilisierbares Indikatormittel, das sich im Behälter im Bereich des transparenten Abschnitts befindet, wobei dieses Indikatormittel eine messbare Veränderung anzeigt als Reaktion auf eine pH-Veränderung in seiner Umgebung, welche durch diesen transparenten Abschnitt nachweisbar ist nachdem es Stoffwechselprodukten von mikrobiellem Wachstum ausgesetzt worden ist, wobei Veränderungen im Indikatormittel von ausserhalb des Behälters durch diesen transparenten Abschnitt überwacht werden können, wodurch mikrobielles Wachstum überwacht werden kann, ohne nach dem Versiegeln in den Behälter einzudringen;
ein Emittermittel zum Emittieren eines Emittersignals, das mit mindestens einer messbaren Eigenschaft dieses Indikatormittels interagiert, wodurch ein Indikatorsignal erzeugt wird, wobei dieses Emittermittel in bezug zu diesem Indikatormittel derart positioniert ist, dass dieses Emittersignal durch den transparenten Abschnitt auf dieses Indikatormittel auftrifft;
ein Nachweismittel, das in bezug auf dieses Indikatormittel derart positioniert ist, um dieses Indikatorsignal von diesem Indikatormittel durch den transparenten Abschnitt zu empfangen, und um ein dementsprechendes Nachweissignal zu erzeugen;
Verarbeitungsmittel zum Empfangen dieses Nachweissignals und zum Verarbeiten dieses Nachweissignals, um die Grösse der messbaren Eigenschaft dieses Indikatormittels zu jeder gegebenen Zeit zu evaluieren, und um die Grösse dieses Signals mit der Grösse zu einer anderen Zeit zu vergleichen und dabei mikrobielles Wachstum in diesem versiegelbaren Behälter zu überwachen, nachdem dieser Behälter versiegelt worden ist.

2. Instrument nach Anspruch 1, worin dieses Verarbeitungsmittel zum Empfangen dieses Nachweissignals ein Schaltmittel ist.

3. Instrument nach Anspruch 1, wobei dieses Emittermittel eine lichtemittierende Diode umfasst.

4. Instrument nach Anspruch 3, wobei dieses Nachweismittel eine Photodiode umfasst, die Reflexionen der Emission dieser lichtemittierenden Diode empfangen kann.

5. Instrument nach Anspruch 3, wobei dieses Verarbeitungsmittel zum Empfangen dieses Nachweissignals eine stabilisierte Stromquelle für diese lichtemittierende Diode umfasst.

6. Instrument nach Anspruch 2, wobei dieses Schaltmittel Rechenmittel umfasst, um dieses Nachweissignal in ausgewählten Zeitintervallen zu empfangen, und um alle Veränderungen der Eigenschaften dieses Nachweissignals während jedem dieser Zeitintervalle zu vergleichen.

7. Instrument nach Anspruch 2, wobei dieses Schaltmittel Rechenmittel umfasst zum periodischen Bemustern dieses Nachweissignals, zum Vergleichen von Proben dieses Nachweissignals, und zum Berechnen der Veränderungsgeschwindigkeit dieser Proben.

8. Instrument nach Anspruch 1, weiter umfassend Mittel zum Nachweisen einer Veränderungsgeschwindigkeit in diesem Nachweissignal, und zum Messen der Dauer dieser Veränderungsgeschwindigkeit.

9. Verfahren zu Nachweisen von mikrobiellem Wachstum in einer Probe, umfassend folgende Schritte:
Vorsehen eines sterilisierbaren Behälters, wobei der Behälter in einer seiner Wände mindestens einen transparenten Abschnitt aufweist, und ein Indikatormittel, das eine messbare Veränderung anzeigt als Reaktion auf eine pH-Veränderung in seiner Umgebung, die sich im Behälter im Bereich des transparenten Abschnitts befindet;
Vorsehen eines Emittermittels zum Emittieren eines Emittersignals, das mit mindestens einer messbaren Eigenschaft dieses Indikatormittels interagiert, wodurch ein Indikatorsignal erzeugt wird, wobei dieses Emittermittel in bezug zu diesem Indikatormittel derart positioniert ist, dass dieses Emittersignal durch den transparenten Abschnitt auf dieses Indikatormittel auftrifft;
Vorsehen eines Nachweismittels, das in bezug zu diesem Indikatormittel derart positioniert ist, um dieses Indikatorsignal von diesem Indikatormittel zu empfangen, und um ein dementsprechendes Nachweissignal zu erzeugen;
Vorsehen von Verarbeitungsmitteln zum Empfangen dieses Nachweissignals und zum Verarbeiten dieses Nachweissignals, um die Grösse der messbaren Eigenschaft dieses Indikatormittels zu evaluieren;
Einführen der Probe in den Behälter unter sterilen Bedingungen; Inkubieren der Probe im Behälter; und
Nachweisen einer Veränderung im oder der Grösse des Nachweissignals, wodurch mikrobielles Wachstum in der versiegelbaren Flasche nachgewiesen wird, nachdem der Behälter versiegelt worden ist.

10. Verfahren nach Anspruch 9, weiter umfassend folgende Schritte:
Empfangen dieses Nachweissignals in ausgewählten Zeitintervallen;
Vergleichen aller Veränderungen in den Eigenschaften dieses Nachweissignals während dieses Zeitintervalls.

11. Verfahren nach Anspruch 9, weiter umfassend folgende Schritte:
Bemustern dieses Nachweissignals;
Vergleichen von aufeinanderfolgenden Proben dieses Nachweissignals; und
Berechnen der Veränderung der Veränderungsgeschwindigkeit zwischen aufeinanderfolgenden Proben.

12. Verfahren nach Anspruch 9, weiter umfassend folgende Schritte:
Nachweisen einer Zunahme der messbaren Eigenschaft; und Messen der Dauer dieser messbaren Eigenschaft, die sich auf diesem erhöhten Niveau befindet.

13. Verfahren zum Bestimmen des Vorhandenseins von Mikroben in einer Probe, umfassend folgende Schritte:
Vorsehen des Instruments von Anspruch 1;
Einführen der Proben in den sterilen Behälter;
Inkubieren der Proben im Behälter;
Ueberwachen des Indikatormittels mit dem Nachweismittel bezüglich einer Veränderung einer messbaren Eigenschaft des Indikatormittels in mindestens einem Zeitintervall;
Berechnen der Veränderungsgeschwindigkeit der messbaren Eigenschaft, wobei eine erste Ableitung der Messung der Eigenschaft bestimmt wird; und
Analysieren der Grösse der ersten Ableitung der messbaren Eigenschaft, um das Vorhandensein von Mikroben in der Probe zu ermitteln.

14. Verfahren zum Nachweisen des Vorhandenseins von Mikroben in einer Probe, umfassend folgende Schritte:
Vorsehen des Instruments von Anspruch 1;
Einführen der Probe in den sterilen Behälter;
Inkubieren der Probe im Behälter;
Ueberwachen des Indikatormittels mit dem Nachweismittel bezüglich einer Veränderung in einer messbaren Eigenschaft des Indikatormittels während mindestens einem Zeitintervall; und
Messen der Grösse der messbaren Eigenschaft, um das Vorhandesein von Mikroben nachzuweisen.

15. Verfahren nach Anspruch 13, weiter umfassend folgende Schritte:
Berechnen der zweiten Ableitung der Messung der messbaren Eigenschaft; und
Analysieren der Grösse der zweiten Ableitung der messbaren Eigenschaft, wodurch das Vorhandensein von Mikroben in der Probe nachgewiesen wird.

16. Verfahren nach Anspruch 15, weiter umfassend folgende Schritte:
Messen der Dauer dieser zweiten Ableitung, wenn diese eine gewisse Grösse aufweist.

17. Instrument nach Anspruch 1, worin das Indikatormittel eine Membran und ein Indikatormedium umfasst, wobei das Indikatormedium eine nachweisbare Veränderung anzeigt als Reaktion auf eine pH-Veränderung in seiner Umgebung.

18. Instrument nach Anspruch 1, worin die Membran an eine Innenwand des Behälters geklebt ist.

19. Instrument nach Anspruch 17, worin das Indikatormittel gegen eine Innenwand des Behälters anliegend angeordnet ist und die Membran des Indikatormittels das Indikatormedium vom Kulturmedium trennt.

20. Instrument nach Anspruch 1, worin das Stoffwechselprodukt CO₂ ist.

21. Instrument nach Anspruch 1, worin das Indikatormedium eine Chemikalie ist, die auf pH reagiert.

22. Instrument nach Anspruch 1, weiter umfassend Mittel zum Nachweisen einer Veränderung der Veränderungsgschwindigkeit.

23. Instrument nach Anspruch 22, das ebenfalls die Dauer dieser Veränderung der Veränderungsgeschwindigkeit misst.

24. Instrument nach Anspruch 1, das ebenfalls die Dauer misst, wobei dieses Nachweissignal eine gegebene Grösse aufweist.

## Revendications

1. Un instrument pour suivre la croissance microbienne dans un échantillon, comprenant:
une cuve qui peut être scellée, stérilisable, présentant une chambre interne dans laquelle on met l'échantillon en culture dans un milieu de culture stérile, la cuve possédant au moins une section transparente;
un moyen indicateur stérilisable placé dans la cuve dans la région de la section transparente, ledit moyen indicateur présentant une variation mesurable en réponse à une variation de pH de son milieu environnant détectable à travers ladite section transparente après exposition aux métabolites de la croissance microbienne, lesdites variations du moyen indicateur pouvant être suivies de l'extérieur de la cuve à travers la section transparente, ce qui permet de suivre la croissance microbienne sans avoir besoin de pénétrer dans la cuve après scellement;
un moyen d'émission pour émettre un signal d'émission qui interagit avec au moins une propriété mesurable dudit moyen indicateur ce qui permet de produire un signal indicateur, ledit moyen d'émission étant placé par rapport audit moyen indicateur de manière que ledit signal de l'émetteur frappe ledit moyen indicateur à travers la section transparente;
un moyen de détection positionné relativement audit moyen indicateur pour recevoir ledit signal de l'indicateur émis par ledit moyen indicateur à travers la section transparente et produire un signal du détecteur correspondant;
des moyens de traitement pour recevoir ledit signal du détecteur et pour traiter ledit signal du détecteur pour évaluer l'importance de la propriété mesurable dudit moyen indicateur à tous moments, et comparer l'intensité dudit signal avec l'intensité à un autre moment et donc suivre la croissance microbienne dans ladite cuve scellée après scellement de ladite cuve.

2. Un instrument selon la revendication 1, dans lequel lesdits moyens de traitement pour recevoir ledit signal du détecteur est un moyen de circuit.

3. Un instrument selon la revendication 1, dans lequel ledit moyen d'émission comprend une diode émettrice de lumière.

4. Un instrument selon la revendication 3, ledit moyen de détection comprenant une photodiode réceptrice à la réflexion de l'émission de ladite diode émettrice de lumière.

5. Un instrument selon la revendication 3, lesdits moyens de traitement pour la réception dudit signal du détecteur comprenant une source de courant stabilisée pour ladite diode émettrice de lumière.

6. Un instrument selon la revendication 2, ledit moyen de circuit comprenant:
des moyens informatiques pour recevoir ledit signal du détecteur à des intervalles de temps choisis et pour comparer toutes variations des caractéristiques dudit signal du détecteur au cours de chacun desdits intervalles de temps.

7. Un instrument selon la revendication 2, ledit moyen de circuit comprenant des moyens informatiques pour l'échantillonnage périodique dudit signal du détecteur afin de comparer les échantillons dudit signal du détecteur et pour calculer la vitesse de variation desdits échantillons.

8. Un instrument selon la revendication 1, comprenant en outre des moyens pour détecter une vitesse de variation dans ledit signal du détecteur et pour mesurer la durée de ladite variation de vitesse.

9. Une méthode pour détecter la croissance microbienne dans un échantillon comprenant les étapes:
d'utilisation d'une cuve stérilisable, la cuve présentant au moins une section transparente dans l'une de ses parois et un moyen indicateur qui présente une variation mesurable en réponse à une variation de pH dans son milieu environnant situé dans la cuve dans la région de la section transparente;
d'utilisation d'un moyen d'émission pour émettre un signal d'émission qui interagit avec au moins une propriété mesurable dudit moyen indicateur ce qui permet de produire un signal indicateur, ledit moyen émetteur étant positionné par rapport audit moyen indicateur de manière à ce que le signal de l'émetteur frappe ledit moyen indicateur à travers la section transparente;
d'utilisation d'un moyen de détection positionné relativement audit moyen indicateur de manière à recevoir ledit signal indicateur dudit moyen indicateur et pour générer un signal de détecteur correspondant;
d'utilisation un moyen de traitement pour recevoir ledit signal du détecteur et pour traiter ledit signal du détecteur afin d'évaluer l'amplitude de la propriété mesurable dudit moyen indicateur;
d'introduction de l'échantillon dans des conditions stériles dans la cuve;
d'incubation de l'échantillon dans la cuve; et
de détection d'une variation dans le, ou l'amplitude du, signal du détecteur ce qui permet de détecter une croissance microbienne dans la cuve scellable après que la cuve ait été scellée.

10. Un procédé selon la revendication 9, comprenant en outre les étapes de:
réception dudit signal du détecteur à intervalles de temps choisis; et
comparaison de toutes variations des caractéristiques dudit signal du détecteur au cours desdits intervalles de temps.

11. Un procédé selon la revendication 9, comprenant en outre les étapes:
d'échantillonnage dudit signal du détecteur;
de comparaison des enregistrements successifs dudit signal du détecteur; et
de calcul de la variation de la vitesse de variation entre deux échantillons successifs.

12. Un procédé selon la revendication 9, comprenant en outre les étapes de:
détection d'une augmentation de la propriété mesurable et
mesure de la durée pendant laquelle ladite propriété mesurable se trouve à ce niveau augmenté.

13. Un procédé de détermination de la présence de microbes dans un échantillon, comprenant les étapes:
d'utilisation de l'instrument selon la revendication 1;
d'introduction de l'échantillon dans la cuve stérile;
incubation de l'échantillon dans la cuve;
de contrôle d'une variation dans une propriété mesurable du moyen indicateur à l'aide du moyens de détection à au moins un intervalle de temps;
de calcul de la vitesse de variation de la propriété mesurable, ce qui permet d'obtenir la dérivée première de la mesure de la propriété; et
d'analyse de l'amplitude de la dérivée première de la propriété mesurable pour établir la présence des microbes dans l'échantillon.

14. Un procédé de détermination de la présence de microbes dans un échantillon comprenant les étapes:
d'utilisation de l'instrument selon la revendication 1;
d'introduction de l'échantillon dans la cuve stérile;
d'incubation de l'échantillon dans la cuve;
de contrôle d'une variation d'une propriété mesurable du moyen indicateur par le moyen de détection à au moins un intervalle de temps et
de mesurer l'amplitude de la propriété mesurable pour établir la présence de microbes dans l'échantillon.

15. Un procédé selon la revendication 13, comprenant en outre les étapes de:
calcul de la dérivée seconde de la mesure de la propriété mesurable; et
analyse de l'amplitude de la dérivée seconde de la propriété mesurable, ce qui permet de prouver la présence de microbes dans l'échantillon.

16. Un procédé selon la revendication 15, comprenant en outre les étapes de:
mesure de la durée pendant laquelle ladite dérivée seconde se trouve à une certaine valeur.

17. Un instrument selon la revendication 1, dans lequel le moyen indicateur comprend une membrane et un milieu indicateur, le milieu indicateur présentant une variation détectable en réponse à une variation de pH de son milieu environnant.

18. Un instrument selon la revendication 1, dans lequel la membrane est scellée sur une paroi interne de la cuve.

19. Un instrument selon la revendication 17, dans lequel ledit milieu indicateur est disposé contre une paroi interne de la cuve et la membrane du moyen indicateur sépare le milieu indicateur du milieu de culture.

20. Un instrument selon la revendication 1, dans lequel le métabolite est CO₂.

21. Un instrument selon la revendication 1, dans lequel le milieu indicateur est un produit chimique sensible au pH.

22. Un instrument selon la revendication 1, comprenant en outre des moyens pour détecter une variation de la vitesse de variation.

23. Un instrument selon la revendication 22, capable de mesurer également la durée de ladite variation de la vitesse de variation.

24. Un instrument selon la revendication 1, capable également de mesurer la durée, ledit signal du détecteur se trouvant à un niveau donné.
